# EUROPEAN PATENT SPECIFICATION

(11) **EP 2 027 846 B1**
(45) Date of publication and mention of the grant of the patent: **12.03.2014**
(21) Application number: 08075613.3
(22) Date of filing: 10.07.2008
(51) Int. Cl.: A61M 39/00, F16L 33/22, F16L 37/02, A61M 39/12, A61H 33/00, A61M 39/10

(54) **Bonding socket for high pressure medical hose**
Bindungssockel für einen medizinischen Hochdruckschlauch
Prise de liaison pour tuyau médical haute pression

(30) Priority: 20.08.2007 US 956729 P
(43) Date of publication of application: 25.02.2009
(73) Proprietor: Atrion Medical Products, Inc., Arab, AL 35016 (US)
(72) Inventor: Kanner, Rowland, W., Guntersville, AL 35976 (US)
(74) Representative: Franks & Co Limited

(56) References cited:
- EP-A- 0 880 978
- WO-A-2007/103464
- DE-A1- 3 521 387
- DE-A1- 4 100 837
- DE-C1- 4 328 122
- DE-U1- 20 303 719
- US-A- 4 137 117
- US-B2- 7 766 876

## Description

### Background

The present invention generally relates to sockets for bonding medical hoses, and more specifically relates to a socket configuration for bonding a small diameter medical hose for use in a high pressure application.

High pressure medical hose (i.e., tubing) is generally made by extruding a first tube form, known as an inner jacket, from an clastomeric resin. Once formed and cooled sufficiently to be self-supporting, this tube form is then wrapped with a reinforcing fiber braid of monofilament fibers. Subsequently, the fiber-wrapped assembly is drawn through a cross die extrusion head which extrudes an outer jacket to the assembly, encapsulating the reinforcement fibers between the jacket layers (i.e., between the inner jacket and the outer jacket). If all goes well, the molten outer jacket material bonds to the inner jacket surface and, to some degree, the reinforcing fibers. However, these bonds are never as strong as the parent materials involved. Since the reinforcing fibers are of different material than the jacket material, the bond between the reinforcement fibers and the outer jacket is weaker than the bond between the inner jacket and the outer jacket. Manufacturers of high pressure reinforced medical hoses constantly struggle to produce a hose which has bonds of sufficient strength to resist high pressure failure modes.

Due to low stiffness of the resin used, the resulting hose is generally quite flexible which suits the conditions under which the hose is to be used. A rather open spacing between the reinforcing fibers of the finished assembly facilitates flexibility while imparting extraordinary tensile and pressure-resisting strength. Due to a reinforcement braid, hoses used on angioplasty inflation devices for example, are capable of withstanding applied internal operating pressures of 1,700 p.s.i. or more before bursting.

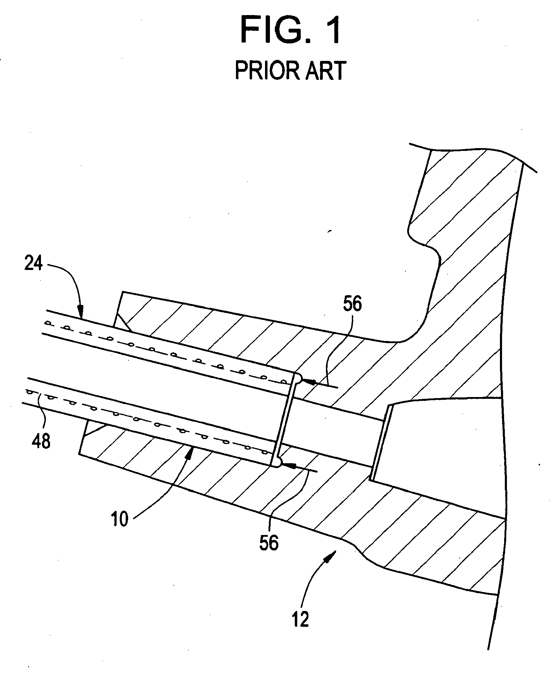

These hoses can be fairly small, having an outer diameter of 0.140 inches and a lumen of less than 0.070 inches. They are most often used on disposable medical devices made of plastic. The pressure-generating medical devices on which these hoses are used must be sufficiently robust in order to withstand high pressures and rough handling. Due to the fact that these hoses have very small passageways, attaching the hose by means of a traditional hose barb form is not practical. Such hose barbs would need to be extraordinarily thin-walled to minimize fluid flow restrictions, rendering them weak and fragile. Therefore, as shown in Figure 1, hoses of this type are typically inserted and bonded into a receiving bore or socket 10 of the pressure device 12. Either solvents or adhesives are utilized to bond the hose to the socket, with solvents being used more often due to the fact that they are easier to apply and handle than adhesives.

When reinforced elastomeric hoses of the type described hereinabove are bonded into receiving sockets of a device, they are prone to suffer from two weaknesses directly attributable to their manufacturing process and overall structure. These weaknesses are aggravated by the traditional hose socket configuration. Specifically, working fluid under pressure within the functioning device can enter locations at the end of the hose where the reinforced fibers provide conduits. If the fibers are not bonded well to the outer jacket, the pressurized fluid begins to bleed along the fibers, and separate the outer jacket from the fibers. The structure of the hose is such that the reinforcing fibers cross one another. As such, their encapsulations at each intersection offer numerous additional conduits for the pressurized fluid. As more fibers become involved in this destructive process, the pressurized fluid begins to inflate the space between the fibers and the jackets until eventually the bond between the inner and the outer jacket fails, and the outer jacket either separates from the inner jacket or it ruptures. Hose failures of this type rob essential working pressure from the medical device and can compromise sterility of the medical procedure as well as destroy the potency of the device.

In EP 0880978 is described a medical tube coupling having a first socket to receive one end of fluid inlet tubing and a second, annulus socket for receiving one end of succeeding tubing for continued fluid flow.

In DE 3521387 is described a hose coupling whereby a hose end is trapped between outer and inner components.

In WO 20071103464 is described a medical connector requiring three components, being a fitting, a coupler, and a member.

In US 4137117 is described a solvent-bonded joint for a pair of members joined at a zone of interference, with the solvent bond in a tapered crevice.

In DE 20303719 is described a connector for medical tubing with radial screws to prevent separation of a barbed, push fit connection between adjacent ends of two tubes to be connected.

In DE 4100837 is described a connector for medical tubing in which a tube end is pushed on to a spigot of the connector, with inturned barbs of the connector adapted to bolt into the external periphery of the tube to prevent withdrawal.

In DE 4328122 is described a two-part coupling for medical tubing with one part engaged over the end of the tubing and having formations adapted to strap behind a collar of the other part, the latter having a spigot entering the tubing and making a tight fit with an interposed sleeve.

In US 7,766,876 is described a gastronomy catheter introducing device comprising a puncturing device.

### Object and Summary

An object of an embodiment of the present invention is to provide an improved medical hose socket, such as for use in high pressure applications.

Briefly, an embodiment of the present invention provides a socket, such as on a medical device for receiving an elastomeric hose or tubing. The hose may be, for example, reinforced medical hose. The socket includes an internal conical feature which is configured to enter an end of the hose when the hose is inserted in the socket. The socket's conical feature compresses the hose and places the hose wall under radial compression, which seals the junction against leakage and increases compression of the hose wall elastomer against its encapsulated reinforcing fiber to prevent introduction of medical fluid along the fiber's path.

The hose according to the present invention is a small diameter medical hose wrapped with reinforcing fibre braid of monofilament fibers to withstand the high pressure used in this application.

### Brief Description of the Drawings

The organization and manner of the structure and operation of the invention, together with further objects and advantages thereof, may best be understood by reference to the following description, taken in connection with the accompanying drawing, wherein:
Figure 1 is an enlarged cross-sectional view of a prior art medical hose socket;
Figure 2 is an enlarged cross-sectional view of a medical hose socket which is in accordance with an embodiment of the present invention;
Figure 3 is similar to Figure 2, but shows a medical hose engaged in the socket;
Figure 4 shows an enlarged perspective view of the device which includes the socket shown in Figures 3 and 4;
Figure 5 is an enlarged cross-sectional view of the device shown in Figure 4;
Figure 6 is an enlarged cross-sectional view of the socket gripping and compressing a hose;
Figure 7 is an enlarged perspective view of the cylindrical extending portion which provides the socket therein;
Figure 8 illustrates the results of some experiments that were conducted with thirty hoses, and is a chart which compares pressure decay for each hose installed on a prior art socket such as shown in Figure 1 to pressure decay for the hose installed on a socket as shown in Figure 2.

### Description

While the invention may be susceptible to embodiment in different forms, there is shown in the drawings, and herein will be described in detail, a specific embodiment of the invention. The present disclosure is to be considered an example of the principles of the invention, and is not intended to limit the invention to that which is illustrated and described herein.

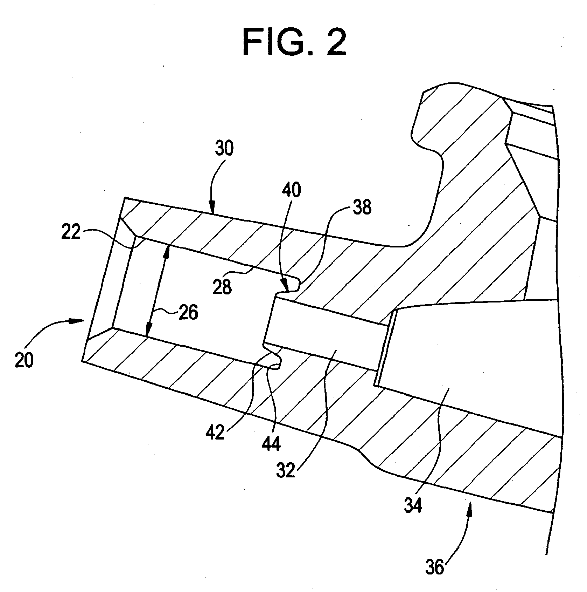

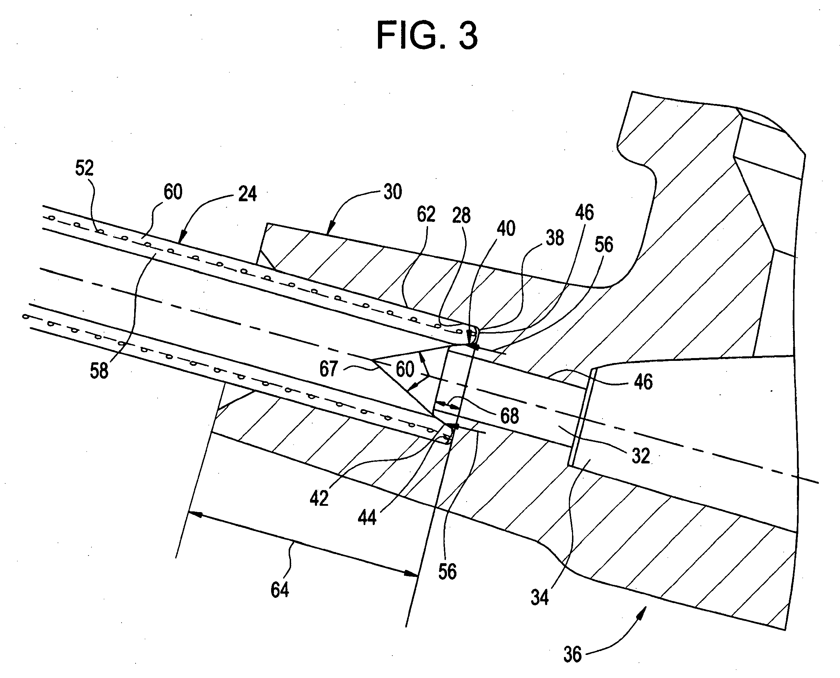

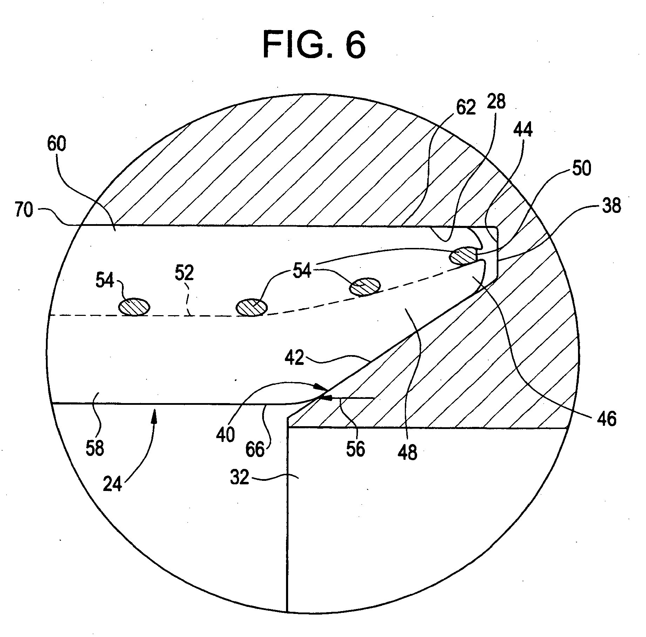

Figure 2 illustrates, in cross-section, a specific embodiment of the present invention. Specifically, Figure 2 illustrates a medical hose socket 20, such as for use in high pressure applications. As shown, much like the hose socket 10 shown in Figure 1, the hose socket 20 shown in Figure 2 includes an opening 22 for receiving a medical hose 24 (See Figures 3 and 6). The medical hose 24 may or may not be a hose which consists of reinforcement fibers encapsulated between two jackets as described hereinabove. Regardless, as will be discussed in more detail below, if a solvent is used to connect the hose 24 to the socket 20, preferably the opening 22 provides an inner diameter (i.e., dimension 26 in Figure 2) that assures a small amount of interference fit with the hose 24 it is to receive (see Figures 3 and 6). On the other hand, if an adhesive is used, preferably a slight clearance is provided between the hose 24 and an internal sidewall 28 to provide space for adhesive to reside.

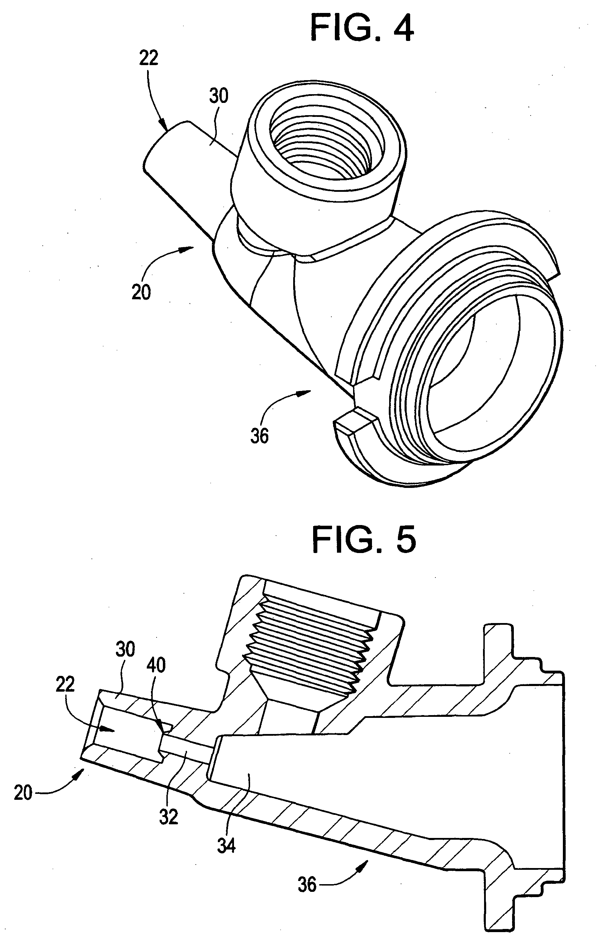

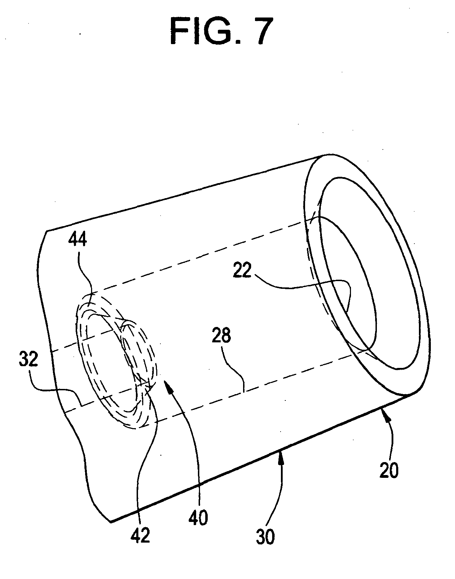

The opening 22 into the socket 20 is provided on a cylindrical extending portion 30, and inside the socket 20 is a conduit 32 which leads to an internal area 34, thereby providing a fluid passageway into the device 36. At the base 38 of the socket 20 is a conical feature or cone 40 (see Figures 2, 3, 5, 6 and 7). The conical feature 40 includes an angled wall 42 proximate the conduit 32. Specifically, inside the cylindrical portion 30 is a longitudinal internal sidewall 28 which ends at a ninety degree angle at a base wall 44 in the socket 20. The base wall 44 intersects the angled wall 42, and the angled wall 42 effectively provides the conical feature 40.

The conical feature 40 in the socket 20 is not a barb, and it functions quite differently. As shown in Figures 3 and 6, the conical feature 40 is configured to enter the end 46 of an inserted hose 24 during the assembly process. As shown, the conical feature 40 serves to compress the hose end 46, thereby placing the hose wall 48 under radial compression. This compression initially utilizes the elastomeric properties of the hose 24 to create a barrier seal between the hose lumen and the conical feature 40 to prevent fluid from reaching reinforcement fiber ends 50 (assuming such a hose 24 is used). In either instance, whether solvent or adhesive bonding is utilized to retain the hose, the space between sidewall 28 of socket 20 and conical feature 40 must be formed such that the smallest end of conical feature 40 is slightly less than the hose lumen in order to allow it to enter the hose and compress the hose wall 48 against sidewall 28 whenever the hose is pressed fully into socket 20. As shown in Figure 6, compression of the hose wall 48 places the jacket bond line or knit line 52, and subsequently each encapsulated fiber 54 under compression in order to raise resistance to pressurized fluid entry, should the first barrier be breached. An additional benefit of the high pressure socket configuration is that, even if a non-reinforced hose is used (i.e., a hose not having internal reinforcing braids), there is less longitudinal force attempting to push the hose out of the socket 20 during pressurization (i.e., in the direction indicated with arrows 56 in Figures 3 and 6). A reduction in force is due to the fact that the cross-sectional area upon which pressure is exerted against the hose 24 is established by an area bordered by intersection of the hose lumen and cone (A1), and this area is always smaller than the cross-sectional area of the entire hose (A2). As such, the longitudinal pressure against the hose is reduced in direct proportion to the two areas ((A1/A2) x system pressure).

The increased retention ability of the socket shown in Figure 2 compared to the socket shown in Figure 1 results from there being less cross-sectional surface area exposed to pressure in the socket combined with a shear resisting connection between the outer hose wall 48 and the socket sidewall 28. This could be solvent based, adhesive based, or even mechanical. While it is true that pressure tending to expand the hose drives the hose wall more solidly into contact with the socket sidewall as long as the seal between the hose lumen and the cone is established during assembly by initial assembly pressure, a separate retaining mechanism to secure the hose wall to the socket wall is still required. Nevertheless, internal expansion due to operational pressures within a device will provide additional retention assistance and compress the elastomeric hose material into more intimate contact with the reinforcing fiber. Experimentation has shown that high internal system pressures will in fact compress the elastomer against the socket wall and prevent flow along reinforcing fibers but for this to happen, a pressure differential must first be created along the fiber's path and that differential depends upon a good initial seal at the cone to prevent fluid loss at pressures below those capable of compressing the hose elastomer tightly against the socket wall. It should be appreciated that compressing pressure will vary with durometer of the hose elastomer.

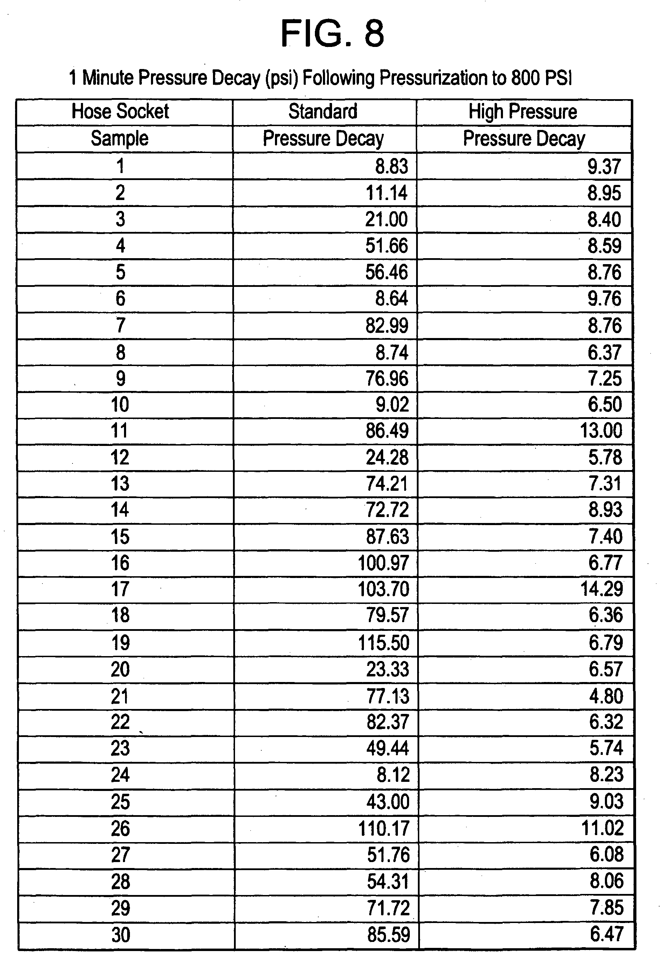

Experiments were conducted to compare pressure loss (decay) performance utilizing a high pressure socket configuration 20 which is in accordance with an embodiment of the present invention (i.e., Figure 2) upon hoses that had previously delaminated under testing with standard hose sockets 10 (i.e., Figure 1). The results were impressive as can be seen by viewing the chart shown in Figure 8 and comparing pressure decay values from 800 p.s.i. for hoses that were bonded to a standard hose socket (i.e., Figure 1) and decay tested and subsequently bonded to the high pressure socket (i.e., Figure 2) and retested. In one instance ("Hose Socket Sample 19" in Figure 8), the same hose that demonstrated 115.50 p.s.i. pressure loss from flow along the reinforcing fiber when bonded to a standard hose socket (i.e., Figure 1) lost only 6.69 p.s.i. due only to expansion when bonded to the high pressure socket (i.e., Figure 2).

Assuming the socket 20 shown in Figure 2 is used with a high pressure reinforced medical hose as described in detail hereinabove (i.e., having an inner jacket 58, an outer jacket 60, and reinforcing braids 54 encapsulated therebetween as shown in Figure 6), the hose 24 is either dipped into or has applied to its end either a solvent or adhesive that is mutually appropriate for the hose material and the device to which the hose will be bonded. Solvent or adhesive is preferably applied to the outer jacket surface 62 for a length along the jacket 60 that is equal to the depth of the hose socket into which the hose 24 will be introduced (i.e., length 64 shown in Figure 3). Hoses treated with solvent are simply pressed firmly into the socket, thereby compressing the compliant hose slightly to assure intimate contact and fusion between socket and hose materials. Pressing the hose 24 firmly in place also allows the conical feature 40 to enter the hose lumen 66 and compress the hose wall 48 between the angled wall 42 and sidewall 28, as insertion force is applied. Friction between the internal sidewall 28 of the socket 20 and the hose exterior 62 serves to hold an inserted hose in place until solvent has fused both pieces together. When adhesives are used to bond the hose to the socket, slight clearance between both parts is required to provide space for adhesive to reside. Therefore, fixturing is required when using adhesive to maintain hose compression against the conical feature until the bond has set. In either case, it is not necessary to apply any solvent or adhesive to hose surfaces that contact the cone. Compression alone is sufficient for the assembly to function as intended.

Female sockets for medical hoses are sized to provide either an interference fit with a hose or clearance relative to the hose as previously described, based upon one's chosen bonding method. Depth of a hose socket for solvent bonding is preferably equal to at least two hose diameters and it may be as much as three. When solvent bonding, assembly interference and a given solvent's flash and diffusion rates place practical limits on socket depth.

In the high pressure hose socket described hereinabove, the included angle (identified with reference numeral 67 in Figure 3) of the cone can vary; however, practical design and manufacturing considerations must be considered since cones rob useful bonding length from sockets. Ideally the cone should be as short as is practical (i.e., dimension 68 in Figure 3) in order to keep hose socket depths to a reasonable level. Cones having low included angles will be longer, thus demanding longer hose sockets. Experiments have shown that cones having approximately 60 degrees of included angle perform well and are reasonably short. Sealing force within this high pressure hose socket results from a combination of applied longitudinal force during hose insertion, circumferential tension generated as the hose stretches over the cone and radial compressive force resulting from the hose wall being compressed in the narrowing space between the cone and the internal wall of the socket.

If the included cone angle were 180 degrees (essentially a flat surface like the base wall 44), only longitudinal compression force would be available to seal. In such a case, no circumferential tension or radial compressive force could be relied upon to assist sealing. With a 180 degree cone, the compression force would need to exceed a calculated value equal to the cross-sectional area of the inner jacket multiplied by the fluid pressure. With cone angles smaller than 180 degrees, the hose expands around the cone as both are pressed together and the circumferential tensile strength of the hose contributes to sealing as does the radial compression force which is generated between the converging walls of the cone and the internal wall of the socket. Therefore, lower cone angles facilitate transition away from a seal reliant upon pure longitudinal compression to one derived from a combination of circumferential tension and radial compression. The net effect of these additional sealing force factors is to reduce the longitudinal compression force required to perfect a seal as cone angles are reduced. Because the amount of longitudinal compression force one must apply to achieve a seal decreases with decreased cone angles, it is believed that cones having greater than a 95 degree included angle would prove less efficient in terms of utilizing longitudinal input forces. This limitation is impacted by the hardness (durometer) of the hose material, its frictional properties against the cone material, and its circumferential strength.

Due to the elastic memory of hose materials, an additional consideration regarding large cone angles is that the force applied to achieve compression against the cone for sealing purposes results in shear at the hose to socket bond line (identified with reference numeral 70 in Figure 6) as the compressed hose 24 attempts to push itself back off a high included angle cone and out of its socket. Reduced cone angles help convert longitudinal installation force into circumferential hose expansion, hose wall compression and friction against the contacting surfaces. With lower cone angles, grip between contact surfaces created by this friction tends to retain the installed hose in place therefore reducing shear force at the bond line.

The specific embodiment described hereinabove provides many advantages some of which have been described hereinabove. While an embodiment of the present invention is shown and described, it is envisioned that those skilled in the art may devise various modifications of the present invention without departing from the scope of the present invention.

## Claims

1. A device comprising a hose (24) and a socket (20), said socket configured to receive a hose at a first end and a rotator at a second opposite end, said socket comprising a cylindrical extending portion (30) and an opening (22), a conical feature (40) down in the opening (22), wherein the cylindrical extending portion (30) has an end which defines the opening and which extends further longitudinally than the conical feature, wherein the conical feature starts at a base wall (44) of the socket and has a vertex which is located within the socket and is surrounded by the cylindrical extending portion of the socket, wherein the socket is configured to compress and grip the hose between the conical feature and the longitudinal internal sidewall (28), **characterized in that** the hose being a small diameter medical hose wrapped with reinforcing fiber (54) braid of monofilament fibers and **in that** the conical feature is providing an included angle (67) being not greater than ninety-five degrees.

2. A device of claim 1, wherein the conical feature (40) is configured to enter an end (46) of the hose (24) when the hose is inserted in the socket (20), wherein the conical feature is configured to compress the hose against the longitudinal internal sidewall (28), and place the hose under radial compression.

3. A device of claim 2, wherein compression of the hose creates a barrier seal between a lumen (66) of the hose (24) and the conical feature (40) to prevent fluid from contacting the end of the hose.

4. A device of claims 1-3, wherein the conduit leads to an internal area, thereby providing a fluid passageway, wherein the angled wall is proximate the conduit, wherein the longitudinal internal sidewall (28) ends at the base wall (44), wherein the base wall intersects the angled wall, and the angled wall provides the conical feature (40).

5. A device of claims 4, wherein the socket (20) is configured to compress and grip the hose (24) between the angled wall and the longitudinal internal sidewall (28).

6. A device of claim 5, wherein the conical feature (40) is configured to enter an end of the hose (24) when the hose is inserted in the socket, wherein the conical feature is configured to compress the hose against the longitudinal internal sidewall (28), and place the hose under radial compression, which tends to keep the hose retained in the socket, even during a high pressure application.

7. A device of claim 6, wherein compression of the hose creates a barrier seal between a lumen (66) of the hose and the conical feature (40) to prevent fluid from contacting the end of the hose.

## Patentansprüche

1. Eine Vorrichtung umfassend einen Schlauch (24) und eine Buchse (20), die Buchse konfiguriert ist, eine Hose an einem Ende und einen Rotor an einem zweiten Gegenende zu empfangen, die Buchse umfassend einen zylindrischen erstreckenden Teil (30) und eine Öffnung (22), eine konische Einrichtung (40) unten in der Öffnung (22), wobei der zylindrische erstreckende Teil (30) ein Ende umfasst, das die Öffnung definiert und das sich weiter longitudinal als die konische Einrichtung erstreckt, wobei die konische Einrichtung an einer Basiswand (44) der Buchse beginnt, und einen Scheitel umfasst, der sich innerhalb der Buchse befindet, und mit dem zylindrischen erstreckenden Teil der Buchse umgegeben ist, wobei die Buchse konfiguriert ist, den Schlauch zwischen der konischen Einrichtung und der inneren Längsseitewand(28) zu komprimieren und zu greifen, **dadurch gekennzeichnet, dass** der Schlauch ein Kleindurchmesser- medischer Schlauch ist, der mit einer Verstärkungsfasertresse (54) von monofaden Fasern eingewickelt ist und dadurch, dass die konische Einrichtung einen einbezogenen Winkel (67) vorsieht, der nicht grösser als 95⁰ ist.

2. Eine Vorrichtung nach Anspruch 1, wobei die konische Einrichtung (40) konfiguriert ist, das Ende (46) des Schlauches (24) zu öffnen, wenn der Schlauch in der Buchse ist, wobei die konische Einrichtung konfiguriert ist, den Schlauch gegen die innere Längsseitewand (28) zu komprimieren, und den Schlauch unten radiale Kompression zu stellen.

3. Eine Vorrichtung nach Anspruch 2, wobei die Schlauchkompression eine Barrierdichtung zwischen einem Lumen (66) des Schlauches (24) und der konischen Einrichtung (40) erzeugt, um Flüssigkeit von Kontakt mit dem Schlauchende zurückzuhalten.

4. Eine Vorrichtung nach einem der Ansprüche 1-3, wobei die Leitung zu einem inneren Bereich führt und damit einen Flüssigkeitsdurchgang erzeugt, wobei die Winkelwand der folgend der Leitung ist, wobei die innere Längsseitewand (28) an der Basiswand (44) endet, wobei die Basiswand die Winkelwand durchschneidet , und die Winkelwand die konische Einrichtung (40) versieht.

5. Eine Vorrichtung nach Anspruch 4, wobei die Buchse (20) konfiguriert ist, den Schlauch zwischen der Winkelwand und der inneren Längsseitewand (28) zu komprimieren and greifen.

6. Eine Vorrichtung nach Anspruch 5, wobei die konische Einrichtung (40) konfiguriert ist, das Ende des Schlauches (24) zu öffnen, wenn der Schlauch in der Buchse ist, wobe die konische Einrichtung konfiguriert ist, den Schlauch gegen die innere Längsseitewand (28) zu komprimieren, und den Schlauch unten radiale Kompression zu stellen, die den Schlauch behielt in der Buchse hält, sogar während einer Hochdrucksverwendung.

7. Eine Vorrichtung nach Anspruch 6, wobei die Schlauchkompression eine Barrierdichtung zwischen einem Lumen (66) des Schlauches und der konischen Einrichtung (40) erzeugt, um Flüssigkeit von Kontakt mit dem Schlauchende zurückzuhalten.

## Revendications

1. Un dispositif comprenant un tuyau (24) et une prise (20), ladite prise étant configurée pour recevoir un tuyau au niveau d'une première extrémité et un rotateur au niveau d'une deuxième extrémité à l'opposée, ladite prise comprenant une portion en extension cylindrique (30) et une ouverture (22), un repère conique (40) dans l'ouverture (22), dans lequel la portion en extension cylindrique (30) possède une extrémité qui définit l'ouverture et qui s'étend longitudinalement davantage que le repère conique, dans lequel le repère conique débute au niveau d'une paroi de base (44) de la prise et possède un sommet qui est situé à l'intérieur de la prise et est entouré par la portion en extension cylindrique de la prise, dans lequel la prise est configurée pour compresser et serrer le tuyau entre le repère conique et la paroi latérale interne longitudinale (28), **caractérisé en ce que** le tuyau est un tuyau médical de faible diamètre enveloppé par une fibre de renforcement (54) tressée de fibres monofilaires et **en ce que** le repère conique fournit un angle d'ouverture (67) n'étant pas supérieur à quatre-vingt- quinze degrés.

2. Un dispositif selon la revendication 1, dans lequel le repère conique (40) est configuré pour pénétrer une extrémité (46) du tuyau (24) lorsque le tuyau est inséré dans la prise (20), dans lequel le repère conique est configuré pour compresser le tuyau contre la paroi latérale interne longitudinale (28) et placer le tuyau sous compression radiale.

3. Un dispositif selon la revendication 2, dans lequel la compression du tuyau crée un joint formant barrière entre un lumen (66) du tuyau (24) et le repère conique (40) pour empêcher du fluide de contacter l'extrémité du tuyau.

4. Un dispositif selon l'une des revendications 1 à 3, dans lequel le conduit mène à une région interne, fournissant ainsi un cheminement pour fluide, dans lequel la paroi inclinée est proche du conduit, dans lequel la paroi latérale interne longitudinale (28) se termine au niveau de la paroi de base (44), dans lequel la paroi de base croise la paroi inclinée, et la paroi inclinée forme le repère conique (40).

5. Un dispositif selon la revendication 4, dans lequel la prise (20) est configurée pour compresser et serrer le tuyau (24) entre la paroi inclinée et la paroi latérale interne longitudinale (28).

6. Un dispositif selon la revendication 5, dans lequel le repère conique (40) est configuré pour pénétrer une extrémité du tuyau (24) lorsque le tuyau est inséré dans la prise, dans lequel le repère conique est configuré pour compresser le tuyau contre la paroi latérale interne longitudinale (28) et placer le tuyau sous compression radiale, qui tend à maintenir le tuyau retenu dans la prise, même à l'occasion d'une application sous haute pression.

7. Un dispositif selon la revendication 6, dans lequel la compression du tuyau crée un joint formant barrière entre un lumen (66) du tuyau (24) et le repère conique (40) pour empêcher du fluide de contacter l'extrémité du tuyau.
